(19) **Europäisches Patentamt / European Patent Office / Office européen des brevets**

(11) **EP 1 546 290 B1**

(12) **EUROPEAN PATENT SPECIFICATION**

(45) Date of publication and mention
of the grant of the patent:
**28.02.2007 Bulletin 2007/09**

(21) Application number: **03766427.3**

(22) Date of filing: **05.08.2003**

(51) Int Cl.:
***C10L 1/18*** (2006.01)

(86) International application number:
**PCT/FI2003/000590**

(87) International publication number:
**WO 2004/013259 (12.02.2004 Gazette 2004/07)**

(54) **A FATTY ACID COMPOSITION, ITS PRODUCTION AND USE**

FETTSûUREZUSAMMENSETZUNG, HERSTELLUNG UND VERWENDUNG DAVON

COMPOSITION D'ACIDES GRAS, SA PRODUCTION ET SON UTILISATION

(84) Designated Contracting States:
**AT BE BG CH CY CZ DE DK EE ES FI FR GB GR LI**

(30) Priority: **05.08.2002 FI 20021446**
**03.12.2002 FI 20022129**

(43) Date of publication of application:
**29.06.2005 Bulletin 2005/26**

(73) Proprietor: **Arizona Chemical**
**1322 CE Almere (NL)**

(72) Inventors:
• **BREWER, Mark**
**NL-1320 AB Almere (NL)**
• **KAUTTO, Torbjörn**
**S-82022 Sandarne (SE)**
• **RAVASKA, Matti**
**FIN-90101 Oulu (FI)**

(74) Representative: **Hjelt, Pia Dorrit Helene et al**
**Borenius & Co Oy Ab**
**Tallberginkatu 2 A**
**00180 Helsinki (FI)**

(56) References cited:
EP-A1- 0 635 558    EP-A1- 0 716 139
EP-A1- 0 739 970    EP-A1- 0 829 527
EP-A2- 1 209 215    WO-A1-97/38965
DE-A1- 4 135 294

• **DUNN R.O. ET AL.: 'Improving the low-temperature properties of alternative diesel duels: vegetable oil-derived methyl esters' JAOCS vol. 73, no. 12, 1996, pages 1719 - 1727, XP002979420**
• **DATABASE WPI Week 199911, Derwent Publications Ltd., London, GB; AN 1999-125637, XP002979421 & JP 11 001 692 A (NIPPON OILS & FATS CO LTD) 06 January 1999**
• **PAOLO BONDIOLI ET AL.: 'Native crambe abyssinica oil and its derivatives as renewable abyssinica oil and its derivatives as renewable lubricants: an approach to improve its quality by chemical and biotechnological processes' INDUSTRIAL CROPS AND PRODUCTS vol. 7, 1998, pages 231 - 238, XP002979422**

EP 1 546 290 B1

**Description**

The present invention relates to a fatty acid composition and to a process for production thereof, and to the use thereof. The fatty acid composition has excellent lubricity performance and good low temperature stability and it is used e.g. as a fuel additive in various types of fuel. Its low temperature properties make it useful also for other uses, such as ore flotation compositions and surfactant compositions. The present invention also relates to a fuel additive and a fuel having good low temperature properties. The preferred fatty acid composition is derived from tall oil.

**Background**

Crude tall oil is recovered from black liquors produced in the kraft pulping process. The composition of crude tall oil is not constant, it varies depending on factors such as cooking, washing, skimming, storage and most of all, the source of wood chips used in the pulping. The tall oil fatty acids are contained in the black liquor in the form of soaps. When the soaps are acidified free fatty acids and resin acids are obtained. The fatty acids and resin acids are normally separated in a separate processing stage by distillation.

Tall oil fatty acids are used in various applications, such as in fuel additive compositions due to their good lubricating properties. They are also used in ore flotation compositions and surfactant compositions.

Many vegetable and animal sources also provide fatty acids which can be used in similar applications.

Fuel additives are used in fuels in order to improve certain aspects of the fuel performance, e.g. lubricity, low temperature properties, or to reduce emissions from the engines. Fuel additives can also be used to allow the use of less polluting fuels in engines without engine damage.

The sulfur content of fuels has been lowered due to environmental legislation aimed at reducing air pollution from sulfur dioxide. The removal of sulfur compounds at the refinery has resulted in the lubricating properties of the fuel being reduced. This in turn has caused increased wear on engine components.

The wear problems in the engines have been solved by adding certain types of additives into the fuel to improve lubricity. Lubricity improvers may be of various types of chemistry including esters, fatty acids, amides and other nitrogen containing material, alcohols etc. Fatty acids have been found to be a cost effective solution. Fatty acid compositions from various sources, such as rapeseed, soya, sunflower, tall oil, have also been used as lubricating agents in fuels.

WO98/04656 discloses a fuel for diesel engines with a sulfur content less than 500 ppm. The fuel comprises at least 20 ppm of a lubricating additive formed by the combination of one monocarboxylic aliphatic hydrocarbon and at least one polycyclic hybrocarbon compound. The fuel contains additive more than 60 ppm when the combination is tall oil.

WO02/20703 discloses a fuel additive composition comprising the reaction product of a mixture of mixed fatty acid esters, a mono or di-(hydroxy alkyl amine) or mixtures thereof and a low temperature property enhancing effective amount of a low molecular weight ester, wherein the reaction mixture has a molar ratio of amine to total ester content in the range from 10.0 to 1.0.

US 3,667,152 discloses a fuel composition comprising a mixture of hydrocarbons boiling in the range from 90 to 625 °F (32 - 329 °C), containing from 0.01 to 0.1 weight percent of tall oil fatty acid. The fatty acid was added in order to provide a fuel having high anti-wear, water separation and thermal stability properties.

WO01/38461 discloses the use of a flow improver for the prevention and/or inhibition of the crystallization of a fatty acid from a composition comprising the fatty acid.

EP 1 209 215 discloses a cold stabilized additive for fuels which contains at least one polar nitrogen containing compound which is effective as paraffin dispergent in the middle distillates. This additive provides the cold stabilizing effect to the mixture. EP 1 209 215 does not disclose fatty acid composition having an improved low temperature stability which does not contain any conventional cold stabilizing additives or any information on the effect of saturated fatty acids, C18;3 fatty acids, C18;2 fatty acids and C18;1 fatty acids on the low temperature properties either.

Jaocs, vol. 73, no. 12, 1996, R.O. Dunn et al., pages 1719-1727 discloses a study on improving the low-temperature properties of alternative diesel fuels. All these alternative diesel fuels are methyl esters from transesterified vegetable oils or animal fats. Thus, they are not based on methyl esters. These alternative diesel fuels are winterised or an additive is added in them.

During the wintertime insufficient low temperature stability of the fuel causes problems in cold climates. Anti-freezing agents and/or low temperature additives have been added to alleviate the problems. Despite the use it has been difficult to find fuel additives which improve lubricating properties and having a sufficient low temperature stability. The consequence of poor low temperature stability in use is the fuel additive separates from the fuel during storage. This may cause plugging resulting in blocking of filters and uneven dosing of the fuel. On the other hand a high amount of additives or many different types of additives in a fuel may cause problems as well.

Thus, there exists a need for fatty acids with improved low temperature properties.

It has now been found that certain fatty acid compositions provide desired low temperature properties. Moreover, it has been found that these fatty acid compositions can be found is tall oil fatty acids. These fatty acids can provide better low temperature properties than those that have been achieved so far in the fatty acid chemistry.

**Summary of the invention**

The present invention relates to a fatty acid composition which fatty acid composition provides low temperature stability to the product that it is added to. The fatty acid composition is preferably used as a fuel additive since it also provides excellent lubricating properties and thus improves the over-all performance of the formulated fuel. As a lubricity improver the composition prevents wear on engines. A parameter which indicates the low temperature stability is the temperature at which the cloud point occurs. The fatty acid composition according to the present invention has a low cloud point. The low temperature properties of the fatty acid composition of the present invention may also be utilized in ore flotation techniques and surfactant compositions.

The fatty acid composition of the invention contains i) more than 10 % C18;3 fatty acids, ii) more than 30 % C18;2 fatty acids, iii) less than 35 % C18;1 fatty acids, iv) less than 3 % saturated fatty acids, and v) more than 90 % of unsaturated fatty acids. Said fatty acids provide improved low temperature stability of the composition and the cloud point of said fatty acid composition is lower than -4 °C.

The preferred fatty acid composition of the present invention is a fatty acid composition having a specific distribution of fatty acids of different chain length and specific amounts of saturated and unsaturated fatty acids.

A fatty acid composition of the present invention contains an effective amount of fatty acids providing improved low temperature stability of the composition. The fatty acids are obtained from tall oil, vegetable fatty acids and/or animal fatty acids.

It has been found that unsaturated fatty acids improve the low temperature stability compared to saturated fatty acids. The low temperature stability of unsaturated fatty acids varies as well. Polyunsaturated fatty acids have better low temperature stability than monounsaturated fatty acids. The preferred fatty acids providing the low temperature stability are C18 fatty acids, preferably unsaturated C18 fatty acids, more preferably polyunsaturated C18 fatty acids. It has been found that especially C18;3 fatty acids, affect the low temperature properties in a beneficial way.

In a preferred embodiment the fatty acid composition contains fatty acids derived from plant sources such as tall oil and/or vegetable oils. A preferred composition contains less than 3 % saturated fatty acids calculated on the total weight of said fatty acids composition and more than 90 %, preferably more than 95 %, more preferably more than 98 %unsaturated fatty acids calculated on the total weight of said fatty acids.

In the preferred embodiment of the invention the fatty acids providing the improved low temperature stability are derived from tall oil. Certain vegetable oils which are high in unsaturated and low in saturated fatty acids are also suitable for use in the invention. Such oils include linseed oil and oil from fish and/or seaweeds

In a tall oil fatty acid composition of the present invention the content of the C18;3 fatty acids is more than 10 %, preferably than 15 %, more preferably more than 20 %, most preferably more than 25 % calculated on the total weight of said fatty acids. In an especially preferred embodiment the C18;3 fatty acid is at least predominantly pinolenic acid.

The amount of the C16 and C 18 saturated fatty acids is preferably as low as possible. The preferred tall oil fatty acid composition of the present invention has the total content of C16;0, C17;0 and C18;0 fatty acids less than 2.2 %, more preferably less than 1 %, most preferably less than 0.5 % calculated on the total weight of said fatty acids.

It is especially important that the content of C18;0 is very low i.e. that the composition contains very little or next to no stearic acid. The stearic acid level is preferably below 0.5%.

In a preferred tall oil fatty acid composition of the present invention the content of C20;0 fatty acids is less than 1 %, preferably less than 0.5 %.

The content of the resin acids in the fatty acid composition of the present invention is low. A tall oil fatty acid composition of the present invention has the content of the resin acids less than 10 %, preferably less than 5 %, more preferably less than 2 %, most preferably less than 1 %.

In a tall oil fatty acid composition of the present invention the content of the C 18;2 fatty acids is more than 30 %, preferably than 40 %, more preferably more than 50 %.

In a tall oil fatty acid composition of the present invention the content of the C18;1 fatty acids is less than 35 %, preferably less than 25 %, more preferably less than 20 %.

Compositions having a high content of 18;3 and a very low content of C 18;0 are preferred since they give very low cloud points. However, it has also been found that the whole composition plays a role in determining the cloud point. The following equation has been developed for use in determining whether any given fatty acid composition is likely to have the desired low temperature characteristics:

Definitions:

[C16;0] means concentration of C 16 saturated fatty acids
[C17;0] means concentration of C17 saturated fatty acids
[C 18;0] means concentration of C 18 saturated fatty acids
[C20;0] means concentration of C20 saturated fatty acids
[C18;1] means concentration of C 18 mono-unsaturated fatty acids
[C 18;2] means concentration of C 18 di-unsaturated fatty acids
[C18;3] means concentration of C18 tri-unsaturated fatty acids
[Resin] means concentration of resin fatty acids

Concentrations are determined by standard ASTM GC method.
Concentration factors

$$A = 6.2$$

$$B = 1.32$$

$$C = 34.5$$

$$D = 0.075$$

$$E = 1.3$$

$$F = -0.27$$

$$G = -5.1$$

$$H = 17$$

Calculation of cloud point factor ($C_{Pfac}$) by equation I

$$Cp_{fac} = A \cdot [C16;0] + B \cdot [C17;0] + C \cdot [C18;0] + D \cdot [C20;0] + E \cdot [C18;1] + F \cdot [C18;2] + G \cdot [C18;3] + H \cdot [Resin]$$

If calculated according to the above mentioned equation, the composition has a low $Cp_{fac}$, i.e. a value below 0.4 the composition is likely to have low temperature properties. A $Cp_{fac}$ value below 0.28 indicates a composition having a cloud point lower the -9 °C, which is considered a very good value for low temperatures. Cloud point factors for typical standard prior art fatty acid compositions are in the order of 1.5 to 0.4.

The cloud point of the tall oil fatty acid composition according to the present invention is low and in a preferred embodiment it is lower than for fatty acid compositions previously known. The cloud point of the fatty acid composition according to the present invention is well below the freezing point of water. In order to be effective, it should be below -4 °C and preferably lower. In a preferred embodiment the cloud point is below -6 °C, preferably below -9 °C. With the composition of the present invention it is possible to provide a cloud point which is lower than -10 °C, preferably lower than -15 °C, more preferably lower than -20 °C.

The present invention also relates to a process for producing a fatty acid composition comprising fatty acids. The process comprises the steps of selecting a crude tall oil having a fatty acid concentration and type, capable of providing low temperature stability for the process and distilling the crude tall oil to provide the desired fatty acid composition containing an effective amount of fatty acids to provide low temperature stability. The specific fatty acid distribution provides good low temperature properties to the composition.

Tall oil from softwood contains 35 to 55 % fatty acids and 20 to 40 % resin acids, while hardwood tall oil contains lower resin acid. The distribution of fatty acids within the tall oil varies with variations in pulp raw material. Thus, for instance trees grown in North America provide a different fatty acid distribution from trees grown in Scandinavia.

The fatty acid composition according to the present invention with improved low temperature performance is produced by the use of raw material selection. The crude tall oil which is used in the process of the present invention is selected based on its fatty acid concentration and type to provide a desired fatty acid composition. It is possible fairly accurately to predict the fatty acid distribution of the distilled tall oil fatty acid composition based on the initial fatty acid concentration and type of the crude tall oil since only minor changes occur during the distillation. Selection of crude tall oil may also include blending of different crude tall oils to provide a desired raw material. In a preferred process of the present invention the crude tall oil is derived from trees grown in a cold climate.

In a preferred crude tall oil for the process of the present invention more than 4 % of the fatty acids are tripleunsaturated fatty acids calculated on the total weight of the fatty acids. A preferred crude tall oil has less than 1 % of the fatty acids which are saturated fatty acids C18 or greater calculated on the total weight of the fatty acids. It is also preferred that the crude tall oil used has 0.3 %, preferably less than 0.2 %, more preferably less than 0.1 % of the fatty acids of the crude tall oil are C 18;0 fatty acids calculated on the total weight of the fatty acids.

The selected crude tall oil is then distilled using a conventional tall oil distilling procedure in order to obtain a fatty acid composition with a specific fatty acid distribution which provides improved low temperature properties.

It is also possible to provide a fatty acid composition of the present invention by blending the appropriate fatty acids derived from different sources.

The fatty acid composition according to the present invention can also be reacted into derivatives, such as esters, amides, and/or amine salts imidazolines, which also have improved low temperature properties. The esters can be mixtures of mono, di, tri esters of glycerol, and esters of pentaeryrithritol, trimethylolpropane, monoethylene glycol, neopentyl glycol and other polyalcohols, and methanol, ethanol, propanol, butanol and 2Ethyl-hexanol and other mono alcohols and/or CnOHm, wherein n = 1-30, m = 1-6. The preferred derivatives are glycerol ester and diethanolamine derivatives.

An ester can be produced from a fatty acid composition containing an effective amount of fatty acids providing improved low temperature stability of the composition and the cloud point of said fatty acid composition is lower than -4 °C. Also a glycerol ester can be produced from fatty acid composition containing an effective amount of fatty acids providing improved low temperature stability of the composition and the cloud point of said fatty acid composition is lower than -4 °C. Such an ester or glycerol ester can be used as a fuel additive.

The fatty acid composition of the present invention is used as a fuel additive as a lubricity improver. The fatty acid composition of the present invention may be added directly to the fuel or it may form a part of a fuel additive package, where such packages are common in the fuel additive industry. Other components that may be present in the fuel additive package are one or more of detergent, cold flow additive, antifoam, static dissipate, antioxidant and other additives used in the art.

The fatty acid composition of the present invention can be used in a fuel additive which is stable at temperature below -4 °C.

The present invention also relates to a fuel containing a fatty acid composition wherein the fuel contains an effective amount of a low temperature stable fatty acid composition lubricity enhancer of the present invention which is stable at temperature below -4 °C. The fatty acid composition according to the present invention is used as a lubricating additive in fuels, such as diesel, gas oil, gasoline, aviation fuel and kerosene and mixtures. The fuel of the present invention can be of low sulfur content, i.e. less than 500 ppm, preferably less than 350 ppm, more preferably less than 50 ppm. The sulfur content of the fuel can be even as low as less than 15 ppm or less than 10 ppm. Typically, 10 to 1000 parts per million (ppm) of the fatty acid composition of the present invention in the fuel is necessary in order to afford improved lubricity to the fuel. It is also used in ore flotation compositions and surfactant compositions.

### Detailed description of the invention

A fatty acid composition according to the present invention is preferably produced by distilling and fractioning crude tall oil to provide a controlled tall oil fatty acid composition. The crude tall oil is distilled using conventional distillation equipment and techniques. The fatty acid composition may also be obtained from vegetable or animal sources.

A fatty acid composition according to the present invention is also produced by blending fatty acids derived from different sources, such as tall oil, vegetable and animal fat. Preferable fatty acids are derived from rapeseed, soy, canola, linseed, tung oil and fish oil. The fatty acids are derived from e.g. distillation fractions or refinery currents. The fatty acid composition of the present invention is made by blending high amount of C18;3 fatty acids and low amount saturated fatty acids with any other fatty acids.

The starting material for the process according to the present invention is selected based on the fatty acid concentration and type of the crude tall oil. By selecting a proper crude tall oil or a blend of tall oils the desired fatty acid composition is obtained. The properties of the fatty acid composition are determined by the amount of the different fatty acids types.

The appropriate crude tall oil is derived from e.g. sources which provide a specific fatty acid content, like trees grown in a special habitat. Different types of trees are grown in different habitat, this determines their characteristic fatty acid concentration and type. The selection of raw material for the crude tall oil affects the quality of the crude tall oil. For example crude tall oil derived from softwood has a different fatty acid composition than crude tall oil derived from hardwood, similarly tree varieties also influence the fatty acid composition.

The crude tall oil used in the present invention is preferably selected based on the tree type. It has been found trees grown in severe cold climate provide a preferred type of crude tall oil to provide a fatty acid composition with improved low temperature stability.

The distillation of the crude tall oil is performed in a conventional distillation system. The distillation system has preferably the steps of dewatering, depitching, heads removal, rosin removal, fatty acid removal and DTO (distilled tall oil) processing. Each of the steps can have single or multiple operations. The distillation process is performed either as a batch or a continuous process.

Each process step is performed using separation techniques which are known as such and which may include wiped, thin and/or falling film evaporators, fractional distillation using different types of column packing. Pressure and/or vacuum, temperature and residence time is also used in the separation techniques.

The crude tall oil is divided into heads, pitch, tall oil rosin, tall oil fatty acid and distilled tall oil. Water and light-volatile oil are distilled first from the crude tall oil. Water and light-volatiles are preferably further processed in other processes. The remaining crude tall oil is distilled further to provide a raw fatty acid distillate by removing high boiling pitch and rosin. The raw fatty acid distillate is fractioned into distilled tall oil and tall oil fatty acids.

"Tall oil" as used in this description and the claims means extractives obtained from wood in kraft pulping. "Crude tall oil" is obtained by acidifying the tall oil soap recovered from the black liquor and it contains fatty acids, resin acids and neutral materials.

"Tall oil fatty acids" means the fatty acids obtained from the crude tall oil by distilling. Tall oil fatty acids in the present invention usually have a chain length C16 to C30. The percentages of the fatty acids in the present description and claims are calculated on the total weight of the fatty acid composition. The significant amount of fatty acid in the description and the claims is 0.1 %. Any content of fatty acid other than the ones mentioned is insignificant in case the concentration is less than 0.1 %.

The fatty acids are designated according to their carbon chain length and number of double bonds according to a standard nomenclature wherein e.g. C18;0 indicates a chain length of 18 carbon atoms and no double bonds while C20;4 indicates a chain length of 20 carbon atoms and 4 double bonds. The position of any double bonds is indicated by numbers e.g. as 18;2-9,12 wherein 9 and 12 indicates the positions of the two double bonds.

"Resin acids" are monocarboxylic diterpene acids, the most common of which has the molecular formula $C_{20}H_{30}O_2$. The resin-based acids can be selected from abietic acid, dihydroabietic acid, dehydroabietic acid, neoabietic acid, pimaric acid, levopimaric acid, palustric acid, isopimaric and other derivatives based on the diterpene structure.

"Unsaponifiables" are neutral substances found in the tall oil which include higher fatty alcohols, esters, plant sterols and some hydrocarbons.

"Low temperature stability" as used in this description and the claims means that the fatty acid composition has a low cloud point. Cloud point is defined as a temperature of a liquid specimen, when a wax crystal structure that is similar in appearance to a cloud is formed upon cooling under prescribed conditions.

The content of the unsaturated and saturated fatty acids is controlled in order to achieve the desired low temperature properties. Especially the content of polyunsaturated fatty acids affects the stability. Therefore, the aim of the invention is to have as much unsaturated fatty acids as possible and only minor amount of saturated fatty acids in the fatty acid composition according to the present invention.

In the present invention it has been found that some crude tall oil contain an exceptionally high level of polyunsaturated C18 fatty acids. When these crude tall oils are distilled with conventional techniques a fatty acid composition is obtained which contains a significant amount of said polyunsaturated C18 fatty acids. The composition thus obtained has a surprisingly effective influence on the low temperature stability of the composition.

It has been found that the content of C18;3 fatty acids is the most critical for the low temperature stability of a fatty acid composition. Therefore the content of C18;3 fatty acids is as high as possible in the composition. An especially preferred C18;3 fatty acid is pinolenic acid since it enhances low temperature stability very effectively. The content C18;2 fatty acids is preferably also high in the composition, since it also influences the stability positively.

Saturated fatty acids have a negative influence on the low temperature stability of a fatty acid composition. Especially the contents of C18;0, C17;0 and C16;0 fatty acids must be low in the fatty acid composition of the present invention in order to have good low temperature stability. Also the content of C20;0 fatty acids is preferably low.

The fatty acid composition of the present invention remains stable well below the freezing point of water. The compositions are stable at temperatures below -4 °C, preferably below -6 °C, more preferably -10 °C. This makes the fatty acid composition effective also at temperatures lower than corresponding prior art products. It is even possible to obtain a fatty acid composition which is stable at temperatures below -15 °C or even below -20 °C.

Low temperature performance of a fatty acid composition is screened using e.g. cloud point, DSC, long term storage at different temperatures (e.g. 5, 0, -15 °C) and cold filtering plugging point (CFPP).

The cloud point may be tested e.g. with an automatic ASTM standard method D5771. The cloud point is defined as a temperature of a liquid specimen, when a wax crystal structure that is similar in appearance to a cloud is formed upon cooling under prescribed conditions. The test is performed by inserting a sample into an apparatus and the sample is cooled according to a cooling profile. The sample is continuously monitored by an optical system for the formation of a crystalline structure. When the crystallization of the wax in the specimen is detected, the temperature is recorded.

Low temperature stability may also be determined by monitoring the appearance of a cooled sample over an extended period of time. A sample is placed in a container which is placed into a cooled environment. The clarity of the sample is visually examined and judged on a predetermined scale on a periodic basis, e.g. daily or weekly.

Differential scanning calorimetry (DSC) is also used to determine the low temperature stability. A sample is subjected to a heating and cooling regime: heat from 25 °C to 100 °C at the rate of 50 °C/min, hold at 100 °C for 2 min, cool from 100 °C to -50 °C at the rate of 10 °C/min, hold at -50 °C for 2 min and heat from -50 °C to 100 °C at 20 °C/min. The exotherms and the endotherms during the heating and cooling are measured with DSC. The sample which has a relatively lower crystallization temperature has a better low temperature stability.

The quality of fuel additives is determined by e.g. the test method IP 450 by Institute of Petroleum. The test method assesses the lubricating property of diesel fuels and fuels which may contain lubricity enhancing additives using a high-frequency reciprocating rig (HFRR). Lubricity fuel additive performance requirements e.g. for Europe is HFRR <460 µm WSD (wear scar diameter).

The fatty and resin acids in a tall oil fractionation product are determined using capillary gas chromatography according to a ASTM standard method D 5974. The amount of the individual fatty acids and resin acids are determined using capillary gas chromatography separation of the volatile methyl esters of these acids.

The fatty acid composition of the present invention may be used as a fuel additive as such or it may be blended with other additives before being added to the fuel. The fatty acid composition is effective as a lubricity improver. The amount of additives added to the fuel may vary depending on many factors such as the type of the fuel and the sulfur content of the fuel. The sulfur content of the fuel is preferably less than 500 ppm, more preferably less than 350 ppm, most preferably less than 50 ppm. The sulfur content of the fuel can be even as low as less than 15 ppm or less than 10 ppm.

Typically, 10 to 1000 parts per million (ppm) of the fatty acid composition of the present invention in the fuel is necessary in order to afford improved lubricity to the fuel.

In the ore recovery processes the fatty acid composition may be substituted for the conventional fatty acid composition used in the froth flotation. This is especially useful in cold climates where the flotation in the wintertime has had problems with the poor stability of conventional compositions at low temperatures such as below -4 °C and especially below -9 °C.

In the following the present invention will be illustrated by some examples which describe some embodiments of the invention.

**Example 1**

Crude tall oil was selected and analyzed by the ASTM standard method D 5974. The contents of different fatty acids were

| | |
|---|---|
| C 16 : 0 | 0.8 |
| C 17 : 0 | 0.4 |
| C 18 : 0 | 0.2 |
| C 18 : 1 | 9.3 |
| C 18 : 2 | 15.2 |
| C 18 : 3 | 5.4 |
| C 20 : 0 | 0.4 |
| resin acids | 34 |

The crude tall oil was distilled in accordance with the present invention to provide a fatty acid composition. The fatty acid composition obtained was analyzed and the content of the fatty acids was:

|  |  |
|---|---|
| C 16 : 0 | 0.1 |
| C 17 : 0 | 0.2 |
| C 18 : 0 | 0.7 |
| C 18 : 1 | 29.7 |
| C 18 : 2 | 45 |
| C 18 : 3 | 14 |
| C 20 : 0 | 0.3 |
| resin acids | <2 |

The cloud point of the fatty acid composition was analyzed with the ASTM standard method D5771. The cloud point was -11 °C. The composition was also analyzed by DSC and the first thermal event on second cooling was -21 °C. The cloud point factor calculated by equation I was 0.14.

The lubricity performance of the fatty acid composition was good and the composition was useful for being added to a fuel as a low temperature stable additive.

Example 2

The crude tall oil of Example 1 was distilled also in a second trial.

The fatty acid composition obtained was analyzed and the content of the fatty acids was:

|  |  |
|---|---|
| C 16 : 0 | 0.2 |
| C 17 : 0 | 0.2 |
| C 18 : 0 | 0.8 |
| C 18 : 1 | 30.2 |
| C 18 : 2 | 47.8 |
| C 18 : 3 | 12.6 |
| C 20 : 0 | 1.0 |
| resin acids | <2 |

The cloud point of the fatty acid composition was analyzed with the ASTM standard method D5771. The cloud point of the fatty acid composition was -15 °C and it was useful as a low temperature stable additive. The cloud point factor calculated according to equation I was 0.25.

Example 3 (Reference Example)

Crude tall oil from American sources was fractionated into a tall oil fatty acid composition with a resin acid content of about 2 %.

The fatty acid composition obtained was analyzed and the the fatty acids comprised:

|  |  |
|---|---|
| C 16 : 0 | 1 |
| C 17 : 0 | 0.3 |
| C 18 : 0 | 2.3 |
| C 18 : 1 | 48.4 |
| C 18 : 2 | 38 |
| C 18 : 3 | 4.8 |
| C 20 : 0 | 0.1 |
| resin acids | <2 |

The cloud point of the fatty acid composition obtained from the American crude tall oil was 7 °C. The content of the C18;3 fatty acid content in oil fatty acid composition was only (4.8 %). The cloud point factor of the composition was found to be 1.48.

**Example 4**

Two fatty acid compositions were tested for their low temperature stability. The first one was the American fatty acid composition of Example 3.

The second fatty acid composition was obtained from trees grown in a cold climate region. It contained the following amounts of fatty acids

| | |
|---|---|
| C 16 : 0 | 0.5 |
| C 17 : 0 | 0.2 |
| C 18 : 0 | 0.5 |
| C 18 : 1 | 24.3 |
| C 18 : 2 | 48.9 |
| C 18 : 3 | 19.5 |
| C 20 : 0 | 0.5 |
| resin acids | <2 |

The cloud point of the cold zone tall oil fatty acid composition was -18 °C, whereas the cloud point of the American tall oil fatty acid composition was 7 °C. DSC analysis of the cold zone composition indicated a first thermal event on second cooling at -28 °C. The cloud point factor was found to be -0.52.

It should be noted that the fatty acid compositions of example 2 and 4 which had excellent low temperature stability (cloud point -12 °C and -18°C, respectively) also showed significantly improved lubricity properties.

**Example 5**

The fatty acid compositions obtained in examples 1, 2 and 4 were tested for their lubricity performance by HFRR (IP450) in < 500 ppm S Diesel fuel. Wear scar diameters in this test are acceptable when they are less than 460$\mu$m. The results are shown below.

| Example | 1 | 4 | 2 |
|---|---|---|---|
| Treat rate (mg/l) | | | |
| Base fuel | 594 | 590 | 590 |
| 50 | 476 | 367 | 350 |
| 100 | 401 | 375 | 375 |
| 200 | 377 | 363 | 374 |

All of the tested tall oil fatty acid compositions were acceptable from a lubricity point of view.

**Example 6**

A crude tall oil obtained from softwood was distilled and was found to provide a fatty acid composition as follows:

| | |
|---|---|
| C 16 : 0 | 0.9 |
| C 17 : 0 | 0.2 |
| C 18 : 0 | 0.3 |
| C 20 : 0 | 0.1 |
| C 18 : 1 | 31.1 |
| C 18 : 2 | 38.2 |
| C 18 : 3 | 10.2 |
| resin acids | 0.5 |

The composition had a cloud point of -15.0°C and an acid value of 195.0. The composition was suitable as for cold climate fuels. Its cloud point factor was 0.03.

The present invention has been illustrated in detail by the above examples. It is evident to those skilled in the art that the invention may be used in many different ways and many different applications.

**Claims**

1.  A fatty acid composition **characterized in that** said composition contains

    i) more than 10 % C 18;3 fatty acids,
    ii) more than 30 % C 18;2 fatty acids,
    iii) less than 35 % C18;1 fatty acids,
    iv) less than 3 % saturated fatty acids, and
    v) more than 90 % of unsaturated fatty acids,

    said fatty acids providing improved low temperature stability of the composition, and that the cloud point of said fatty acid composition is lower than -4 °C.

2.  A fatty acid composition according to claim 1 **characterized in that** said fatty acids are derived from plant sources.

3.  A fatty acid composition according to claim 1 or 2 **characterized in that** said fatty acids are derived from tall oil or vegetable sources.

4.  A fatty acid composition according to claim 1, 2 or 3 **characterized in that** the composition contains less than 1.5 % saturated fatty acids and more than 90 %, preferably more than 95 %, more preferably more than 98 % unsaturated fatty acids.

5.  A fatty acid composition according to claim 4 **characterized in that** the content of the C18;3 fatty acids is more than 15 %, preferably more than 20 %, more preferably more than 25 %.

6.  A fatty acid composition according to claim 5 **characterized in that** said C18;3 fatty acid is pinolenic acid.

7.  A fatty acid composition according to claim 4 or 5 **characterized in that** the total content of C16;0, C17;0 and C18;0 fatty acids is less than 2.2 %, more preferably less than 1 %, most preferably less than 0.5 %,

8.  A fatty acid composition according to claim 4 **characterized in that** the content of C20;0 fatty acids is less than 1 %, preferably less than 0.5 %.

9.  A fatty acid composition according to claim 4 **characterized in that** the content of the resin acids is less than 5 %, preferably less than 2 %, more preferably less than 1 %.

10. A fatty acid composition according to claim 4 **characterized in that** the content of the C18;2 fatty acids is more than 40 %, preferably more than 50 %.

11. A fatty acid composition according to claim 4 **characterized in that** the content of the C18;1 fatty acids is less than 25 %, preferably less than 20 %.

12. A fatty acid composition according to any one of the preceding claims **characterized in that** the composition contains more than 10 %, preferably more than 15 % C18;3 fatty acids and more than 30 %, preferably more than 40 % C 18;2 fatty acids and less than 1 %, preferably less than 0.5 % C18;0 fatty acids and less than 2 %, preferably less than 1 % resin acids and the total of saturated fatty acids is less than 1.5 %.

13. A fatty acid composition according to any one of the preceding claims having a cloud point factor below 0.28 calculated according to the equation I $Cp_{fac}$ = A·[C16;0] + B·[C17;0] + C·[C18;0] + D·[C20;0] + E·[C18;1 + F·[C18;2] + G·[C18;3] + H·[Resin], wherein [16;0] means concentration of C16 saturated fatty acids, [C 17;0] means concentration of C17 saturated fatty acids, [C18;0] means concentration of C18 saturated fatty acids, [C20;0] means concentration of C20 saturated fatty acids, [C18;1] means concentration of C18 mono-unsaturated fatty acids, [C 18;2] means concentration of C 18 di-unsaturated fatty acids, [C18;3] means concentration of C18 tri-unsaturated fatty acids, [Resin] means concentration of C16 resin fatty acids and concentration factors are A = 6.2, B = 1.32, C = 34.5, D = 0.075, E = 1.3, F = -0.27, G = -5.1 and H = 17.

14. Use of a fatty acid composition according to any one of the preceding claims for producing an ester of mono alcohol or polyalcohol.

**15.** Use according to claim 14, wherein said ester is a glycerol ester.

**16.** A process for producing a fatty acid composition according to claim 1 **characterized in that** said process comprises the steps of

    i) selecting a crude tall oil having a fatty acid concentration and type capable of providing low temperature stability, and
    ii) distilling said crude tall oil to provide a fatty acid composition containing an effective amount of tall oil fatty acids providing low temperature stability.

**17.** A process according to claim 16 **characterized in that** selecting includes blending of different crude tall oils.

**18.** A process according to claim 16 **characterized in that** said crude tall oil is derived from trees grown in a cold climate.

**19.** A process according to claim 16 **characterized in that** more than 4 % of the fatty acids of the crude tall oil are triple unsaturated fatty acids.

**20.** A process according to claim 16 **characterized in that** less than 1 % of the fatty acids of the crude tall oil are saturated fatty acids of C18 or greater.

**21.** A process according to claim 13 **characterized in that** less than 0.3 %, preferably less than 0.2 %, more preferably less than 0.1 % of the fatty acids of the crude tall oil are C 18;0 fatty acids.

**22.** Use of a fatty acid composition according to claim 1 as a fuel additive.

**23.** Use of a fatty acid composition according to claim 1 as a lubricity improver in fuel.

**24.** Use according to claim 23 **characterized in that** said lubricity improver forms a part of a fuel additive package containing other additives.

**25.** Use according to claim 24 **characterized in that** said other additives are one or more of detergent, cold flow additive, antifoam, static dissipate and/or antioxidant.

**26.** A fuel containing a fatty acid additive **characterized in that** said fuel contains an effective amount of a low temperature stable fatty acid lubricity enhancer according to claim 1 which is stable at temperature below -4 °C.

**27.** A fuel according to claim 26 **characterized in that** said fuel is diesel, gas oil, gasoline, aviation fuel or kerosene, or a mixture thereof.

**28.** A fuel according to claim 26 **characterized in that** sulfur content of said fuel is less than 500 ppm, preferably less than 350 ppm, more preferably less than 50 ppm, more preferably less than 15 ppm, most preferably less than 10 ppm.

**29.** A fuel according to claim 26 **characterized in that** said fuel contains 10 to 1000 ppm of said fatty acid lubricity enhancer.

**Patentansprüche**

**1.** Fettsäurezusammensetzung, **dadurch gekennzeichnet, daß** die Zusammensetzung enthält:

    i) mehr als 10 % C18;3 Fettsäuren,
    ii) mehr als 30 % C18;2 Fettsäuren,
    iii) weniger als 35 % C18;1 Fettsäuren,
    iv) weniger als 3 % gesättigte Fettsäuren und
    v) mehr als 90 % ungesättigte Fettsäuren,

wobei die Fettsäuren eine verbesserte Niedertemperaturstabilität der Zusammensetzung bereitstellen, und **dadurch**, daß der Trübungspunkt der Fettsäurezusammensetzung niedriger als -4°C ist.

2. Fettsäurezusammensetzung gemäß Anspruch 1, **dadurch gekennzeichnet, daß** die Fettsäuren aus Pflanzenquellen stammen.

3. Fettsäurezusammensetzung gemäß Anspruch 1 oder 2, **dadurch gekennzeichnet, daß** die Fettsäuren aus Tallöl oder Gemüsequellen stammen.

4. Fettsäurezusammensetzung gemäß Anspruch 1, 2 oder 3, **dadurch gekennzeichnet, daß** die Zusammensetzung weniger als 1,5 % gesättigte Fettsäuren und mehr als 90 %, bevorzugt mehr als 95 %, bevorzugter mehr als 98 %, ungesättigte Fettsäuren enthält.

5. Fettsäurezusammensetzung gemäß Anspruch 4, **dadurch gekennzeichnet, daß** der Gehalt der C18;3 Fettsäuren mehr als 15 %, bevorzugt mehr als 20 %, bevorzugter mehr als 25 %, beträgt.

6. Fettsäurezusammensetzung gemäß Anspruch 5, **dadurch gekennzeichnet, daß** die C18;3 Fettsäure Pinolensäure ist.

7. Fettsäurezusammensetzung gemäß Anspruch 4 oder 5, **dadurch gekennzeichnet, daß** der Gesamtgehalt an C16;0, C17;0 und C18;0 Fettsäuren weniger als 2,2 %, bevorzugter weniger als 1 %, am bevorzugtesten weniger als 0,5 % beträgt.

8. Fettsäurezusammensetzung gemäß Anspruch 4, **dadurch gekennzeichnet, daß** der Gehalt an C20;0 Fettsäuren weniger als 1 %, bevorzugt weniger als 0,5 %, beträgt.

9. Fettsäurezusammensetzung gemäß Anspruch 4, **dadurch gekennzeichnet, daß** der Gehalt der Harzsäuren weniger als 5 %, bevorzugt weniger als 2 %, bevorzugter weniger als 1 %, beträgt.

10. Fettsäurezusammensetzung gemäß Anspruch 4, **dadurch gekennzeichnet, daß** der Gehalt der C18;2 Fettsäuren mehr als 40 %, bevorzugt mehr als 50 %, beträgt.

11. Fettsäurezusammensetzung gemäß Anspruch 4, **dadurch gekennzeichnet, daß** der Gehalt der C18;1 Fettsäuren weniger als 25 %, bevorzugt weniger als 20 %, beträgt.

12. Fettsäurezusammensetzung gemäß einem der vorangehenden Ansprüche, **dadurch gekennzeichnet, daß** die Zusammensetzung mehr als 10 %, bevorzugt mehr als 15 %, C18;3 Fettsäuren und mehr als 30 %, bevorzugt mehr als 40 %, C18;2 Fettsäuren und weniger als 1 %, bevorzugt weniger als 0,5 %, C18;0 Fettsäuren und weniger als 2 %, bevorzugt weniger als 1 %, Harzsäuren enthält und die Gesamtmenge der gesättigten Fettsäuren weniger als 1,5 % beträgt.

13. Fettsäurezusammensetzung gemäß einem der vorangehenden Ansprüche mit einem Trübungspunktfaktor unter 0,28, berechnet gemäß der Gleichung I $Cp_{fac} = A \cdot [C16;0] + B \cdot [C17;0] + C \cdot [C18;0] + D \cdot [20;0] + E \cdot [18;1] + F \cdot [C18;2] + G \cdot [C18;3] + H \cdot [Harz]$, worin [C16;0] die Konzentration der C16 gesättigten Fettsäuren bedeutet, [C17;0] die Konzentration der C17 gesättigten Fettsäuren bedeutet, [C18;0] die Konzentration der C18 gesättigten Fettsäuren bedeutet, [C20;0] die Konzentration der C20 gesättigten Fettsäuren bedeutet, [C18;1] die Konzentration der C18 mono-ungesättigten Fettsäuren bedeutet, [C18;2] die Konzentration der C18 di-ungesättigten Fettsäuren bedeutet, [C18;3] die Konzentration der C18 tri-ungesättigten Fettsäuren bedeutet, [Harz] die Konzentration der C16 Harzfettsäuren bedeutet, und die Konzentrationsfaktoren A = 6,2, B = 1,32, C = 34,5, D = 0,075, E = 1,3, F = -0,27, G = -5,1 und H = 17 sind.

14. Verwendung einer Fettsäurezusammensetzung gemäß einem der vorangehenden Ansprüche zur Herstellung eines Monoalkohol- oder Polyalkoholesters.

15. Verwendung gemäß Anspruch 14, worin der Ester ein Glycerinester ist.

16. Verfahren zur Herstellung einer Fettsäurezusammensetzung gemäß Anspruch 1, **dadurch gekennzeichnet, daß** das Verfahren die Schritte umfaßt:

   i) Auswählen eines Rohtallöls mit einer Fettsäurekonzentration und einem Typ, das in der Lage ist, Niedertemperaturstabilität bereitzustellen, und

ii) Destillieren des Rohtallöls, um eine Fettsäurezusammensetzung enthaltend eine wirksame Menge der Tallölfettsäuren, die Niedertemperaturstabilität bereitstellen, bereitzustellen.

**17.** Verfahren gemäß Anspruch 16, **dadurch gekennzeichnet, daß** das Auswählen das Mischen von verschiedenen Rohtallölen einschließt.

**18.** Verfahren gemäß Anspruch 16, **dadurch gekennzeichnet, daß** das Rohtallöl aus im kalten Klima gewachsenen Bäumen stammt.

**19.** Verfahren gemäß Anspruch 16, **dadurch gekennzeichnet, daß** mehr als 4 % der Fettsäuren des Rohtallöls dreifach ungesättigte Fettsäuren sind.

**20.** Verfahren gemäß Anspruch 16, **dadurch gekennzeichnet, daß** weniger als 1 % der Fettsäuren des Rohtallöls gesättigte Fettsäuren von C18 oder größer sind.

**21.** Verfahren gemäß Anspruch 13, **dadurch gekennzeichnet, daß** weniger als 0,3 %, bevorzugt weniger als 0,2 %, bevorzugter weniger als 0,1 %, der Fettsäuren des Rohtallöls C18;0 Fettsäuren sind.

**22.** Verwendung einer Fettsäurezusammensetzung gemäß Anspruch 1 als Kraftstoffadditiv.

**23.** Verwendung einer Fettsäurezusammensetzung gemäß Anspruch 1 als Schmierfähigkeitsverbesserer in Kraftstoff.

**24.** Verwendung gemäß Anspruch 23, **dadurch gekennzeichnet, daß** der Schmierfähigkeitsverbesserer einen Teil eines Kraftstoffadditivgebindes enthaltend andere Additive bildet.

**25.** Verwendung gemäß Anspruch 24, **dadurch gekennzeichnet, daß** die anderen Additive ein oder mehrere von Detergenzien, Kaltfließfähigkeitsadditiv, Schäumverhinderungsmittel, Antistatikmittel und/oder Antioxidantien sind.

**26.** Kraftstoff enthaltend ein Fettsäureadditiv, **dadurch gekennzeichnet, daß** der Kraftstoff eine wirksame Menge eines niedertemperaturstabilen Fettsäureschmierfähigkeitsverstärkers gemäß Anspruch 1 enthält, der bei einer Temperatur unter -4°C stabil ist.

**27.** Kraftstoff gemäß Anspruch 26, **dadurch gekennzeichnet, daß** der Kraftstoff Diesel, Gasöl, Benzin, Flugzeugkraftstoff oder Kerosin oder eine Mischung davon ist.

**28.** Kraftstoff gemäß Anspruch 26, **dadurch gekennzeichnet, daß** der Schwefelgehalt des Kraftstoffs weniger als 500 ppm, bevorzugt weniger als 350 ppm, bevorzugter weniger als 50 ppm, bevorzugter weniger als 15 ppm, am bevorzugtesten weniger als 10 ppm beträgt.

**29.** Kraftstoff gemäß Anspruch 26, **dadurch gekennzeichnet, daß** der Kraftstoff 10 bis 1.000 ppm des Fettsäureschmierfähigkeitsverstärkers enthält.

**Revendications**

**1.** Composition d'acides gras **caractérisée en ce que** ladite composition contient

i) plus de 10% d'acides gras C18;3,
ii) plus de 30% d'acides gras C18;2
iii) plus de 35% d'acides gras C18;1
iv) moins de 3% d'acides gras saturés, et
v) plus de 90% d'acides gras insaturés,

lesdits acides gras offrant à la composition une meilleure stabilité à basse température, et **en ce que** le point de rosée de ladite composition d'acides gras est plus faible que -4°C.

**2.** Composition d'acides gras selon la revendication 1 **caractérisée en ce que** lesdits acides gras sont dérivés de sources de plantes.

**3.** Composition d'acide gras selon la revendication 1 ou 2 **caractérisée en ce que** lesdits acides gras sont dérivés de tall oil ou de sources végétales.

**4.** Composition d'acide gras selon la revendication 1, 2 ou 3 **caractérisée en ce que** la composition contient moins de 1,5% d'acide gras saturé et plus de 90%, de préférence plus de 95%, et mieux plus de 98% d'acides gras insaturés.

**5.** Composition d'acides gras selon la revendication 4, **caractérisée en ce que** la teneur en acides gras C18;3 est supérieure à 15%, de préférence supérieure à 20%, et mieux supérieure à 25%.

**6.** Composition d'acides gras selon la revendication 5, **caractérisée en ce que** ledit acide gras C18;3 est de l'acide pinolénique.

**7.** Composition d'acides gras selon la revendication 4 ou 5, **caractérisée en ce que** la teneur totale en acide gras C16;0, C17;0 et C18;0 est inférieure à 2,2%, mieux inférieure à 1%, et encore mieux inférieure à 0,5%,

**8.** Composition d'acides gras selon la revendication 4, **caractérisée en ce que** la teneur en acides gras C20;0 est inférieure à 1%, de préférence inférieure à 0,5%.

**9.** Composition d'acides gras selon la revendication 4 **caractérisée en ce que** la teneur des acides de la résine est inférieure à 5%, de préférence inférieure à 2%, et mieux inférieure à 1%.

**10.** Composition d'acides gras selon la revendication 4, **caractérisée en ce que** la teneur en acide gras C18;2 est supérieure à 40%, de préférence supérieure à 50%.

**11.** Composition d'acides gras selon la revendication 4, **caractérisée en ce que** la teneur en acide gras C18;1 est inférieure à 25%, de préférence inférieure à 20%.

**12.** Composition d'acides gras selon l'une quelconque des revendications précédentes, **caractérisée en ce que** la composition contient plus de 10%, de préférence plus de 15% d'acides gras C18;3 et plus de 30%, de préférence plus de 40% d'acides gras C18;2 et moins de 1%, de préférence moins de 0,5% d'acides gras C18;0 et moins de 2%, de préférence moins de 1% d'acides de résine et la teneur totale des acides gras saturés est inférieure à 1,5%.

**13.** Composition d'acides gras selon l'une quelconque des revendications précédentes ayant un facteur de point de rosée en dessous de 0,28 en calculant selon l'équation I $Cp_{fac}$ = A.[C16;0] + B.[C17;0] + C.[C18,0] + D.[C20,0] + E. [C18;1] + F. [C18;2] + G. [C18;3] + H.[Résine]; où [C16;0] signifie la concentration des acides gras saturés C16, [C17;0] signifie la concentration des acides gras saturés C17, [C18;0] signifie la concentration des acides gras saturés C18, [C20;0] signifie la concentration des acides gras saturés C20, [C18;1] signifie la concentration des acides gras mono-insaturés, [C18;2] signifie la concentration des acides gras di-insaturés, [C18;3] signifie la concentration des acides gras tri-insaturés C18, [Résine] signifie la concentration des acides gras des résines C16 et les facteurs de concentration sont A=6,2, B=1,32, C=34,5, D=0,075, E=1,3, F=-0,27, G=-5,1 et H=17.

**14.** Utilisation d'une composition d'acides gras selon l'une quelconque des revendications précédentes pour la production d'un ester d'un mono alcool ou d'un polyalcool.

**15.** Utilisation selon la revendication 14, où ledit ester est un ester de glycérol.

**16.** Procédé de production d'une composition d'acide gras selon la revendication 1 **caractérisé en ce que** ledit procédé comprend les étapes de

   i) sélectionner un tall oil brut ayant une concentration en acides gras et un type capables de donner une stabilité à basse température, et
   ii) distiller ledit tall oil brut pour donner une composition d'acides gras contenant une quantité efficace d'acides gras de tall oil donnant la stabilité à basse température.

**17.** Procédé selon la revendication 16 **caractérisé en ce que** la sélection comprend le mélange de différents tall oils bruts.

**18.** Procédé selon la revendication 16 **caractérisé en ce que** ledit tall oil brut est dérivé d'arbres poussant dans un climat froid.

**19.** Procédé selon la revendication 16 **caractérisé en ce que** plus de 4% des acides gras du tall oil brut sont des acides gras insaturés triples.

**20.** Procédé selon la revendication 16 **caractérisé en ce que** moins de 1% des acides gras du tall oil brut sont des acides gras saturés C18 ou plus.

**21.** Procédé selon la revendication 13 **caractérisé en ce que** moins de 0,3%, de préférence moins de 0,2%, mieux moins de 0,1% des acides gras du tall oil brut sont des acides gras C18;0.

**22.** Utilisation d'une composition d'acides gras selon la revendication 1 comme additif de carburant.

**23.** Utilisation d'une composition d'acides gras selon la revendication 1 comme agent améliorant l'onctuosité dans un carburant.

**24.** Utilisation selon la revendication 23 **caractérisée en ce que** l'agent améliorant l'onctuosité fait partie d'un ensemble additif de carburant contenant d'autres additifs.

**25.** Utilisation selon la revendication 24 **caractérisée en ce que** lesdits autres additifs sont un ou plusieurs d'un détergent, d'un additif d'écoulement à froid, d'un anti-mousse, d'un dissipateur statique et/ou d'un antioxydant.

**26.** Carburant contenant un additif d'acide gras **caractérisé en ce que** ledit carburant contient une quantité efficace d'un agent améliorant l'onctuosité d'acides gras stables à basse température selon la revendication 1 qui est stable à une température inférieure à -4°C.

**27.** Carburant selon la revendication 26 **caractérisé en ce que** ledit carburant est du Diesel, du gas-oil, de l'essence, du carburant pour l'aviation ou du kérosène, ou un mélange.

**28.** Carburant selon la revendication 26 **caractérisé en ce que** la teneur en soufre dudit carburant est inférieure à 500 ppm, de préférence inférieur à 350 ppm, mieux inférieur à 50 ppm, et encore mieux inférieur à 15 ppm, et plus de préférence inférieur à 10 ppm.

**29.** Carburant selon la revendication 26 **caractérisé en ce que** ledit carburant contient 10 à 1000 ppm dudit agent améliorant l'onctuosité à base d'acides gras.